Europäisches Patentamt

European Patent Office

Office européen des brevets

(11) Veröffentlichungsnummer : **0 101 779**
**B1**

(19)

(12) # EUROPÄISCHE PATENTSCHRIFT

(45) Veröffentlichungstag der Patentschrift :
**16.06.87**

(21) Anmeldenummer : **82890123.1**

(22) Anmeldetag : **26.08.82**

(51) Int. Cl.⁴ : **C 08 L 83/04**, A 61 K 6/10,
C 08 K 7/22, B 29 C 67/24//
C08J9/32

(54) **Verwendung von Massen zur Herstellung oder Unterfütterung von Zahnprothesen und Verfahren zur Herstellung der Massen.**

(43) Veröffentlichungstag der Anmeldung :
**07.03.84 Patentblatt 84/10**

(45) Bekanntmachung des Hinweises auf die Patenterteilung : **16.06.87 Patentblatt 87/25**

(84) Benannte Vertragsstaaten :
**AT CH FR GB LI NL**

(56) Entgegenhaltungen :
**EP-A- 0 029 021**
**DE-B- 1 629 381**
**DE-B- 2 036 720**
**DE-B- 2 519 910**
**GB-A- 2 026 000**
**US-A- 3 850 864**
**US-A- 4 108 806**

(73) Patentinhaber : **DISTROPAT AG**
**Gartenstrasse 2**
**CH-6300 Zug (CH)**

(72) Erfinder : **Schaefer, Philipp**
**Oberstrasse 16**
**D-3000 Hannover 1 (DE)**
Erfinder : **Rolf, Matthias**
**Immanuel-Kant Strasse 50**
**D-3280 Bad Pyrmont (DE)**

(74) Vertreter : **Boeckmann, Peter, Dipl.-Ing. et al**
**Patentanwälte Dipl.-Ing. Peter Boeckmann, Dipl.-Ing.**
**Leo Brauneiss Strohgasse 10**
**A-1030 Wien (AT)**

## Beschreibung

Die Erfindung betrifft die vervendung von Massen zur Herstellung oder Unterfütterung von Zahnprothesen, bestehend aus einem Silikon, das zunächst flüssig oder pastös bzw. knetbar sich durch Zusetzen eines Härters nach einer bestimmten Zeit kautschukartig verfestigt, und aus dem Silikon beigemengten druckelastischen Mikrohohlkugeln, deren dünne Hülle aus Vinylidenchlorid-Copolymerisat besteht und deren Inneres ein Gas enthält. Ferner betrifft die Erfindung ein Verfahren zur Herstellung einer solchen Masse.

In der Zahnmedizin sind bereits Massen für die Herstellung eines Abdruckes, wie er zur Anfertigung von Prothesen erforderlich ist, aber auch für die Herstellung von Unterfütterungen für solche Prothesen, um sie dem sich ändernden Kiefer anzupassen, bekannt, welche aus einem Silikon bestehen, das zunächst flüssig oder pastös bzw. knetbar ist und sich durch Zusetzen eines Härters nach einer bestimmten Zeit kautschukartig verfestigt.

Unter dem Begriff « Silikon » werden synthetische polymere Verbindungen verstanden, in denen Siliciumatome über Sauerstoffatome verknüpft und die restlichen Valenzen des Siliciums durch Kohlenwasserstoffreste abgesättigt sind. Nähere Angaben über solche Silikone finden sich in Römpps Chemie-Lexikon, 7. Auflage/1975, Franckh-sche Verlagshandlung W. Keller & Co., Stuttgart, Band 5, Spalten 3223 ff.

Die heute üblichen knetbaren Silikonmassen, welche in der Zahnmedizin zur Herstellung von Abdrücken verwendet werden, besitzen den Nachteil einer hohen Dichte und eines schlechten Fließverhaltens. Die Verformung muß daher mit erheblichem Druck erfolgen und es findet dennoch eine unvollkommene Feinabformung statt, da diese Massen in kleine Hohlräume nicht einzudringen vermögen. Aus diesem Grunde werden die knetbaren Silikonmassen auch nur zur Anfertigung eines Vorabdruckes verwendet, der noch einer Korrektur bedarf. Hiezu wird eine dünnflüssige Silikonabformmasse auf den Vorabdruck appliziert und dann der hergestellte Vorabdruck nochmals an die abzuformende Stelle des Kiefers angepaßt. Dies bedeutet eine zweimalige Belästigung des Patienten, wobei dieser auch zweimal mit dem Härter in Berührung kommt, welcher auf der Haut, insbesondere auf Schleimhäuten, Reizungen verursachen kann.

Auch für den Mediziner sind die Arbeiten mit der bekannten Masse schwierig, da einerseits diese Masse knetartig sein muß, damit sie leicht und schnell in den Abdrucklöffel gespachtelt bzw. gedrückt werden kann und damit sie beim Abdrücken nicht in den Rachen fließt, andererseits aber auch feinste Strukturen abgeformt werden sollen. Da die Masse nicht flüssig sein darf, benötigt das Einarbeiten des Härters viel Geschick und Kraft, wobei noch hinzukommt, daß hiefür wegen des gewünschten raschen Aushärtens der Masse wenig Zeit zur Verfügung steht.

Es sind auch Massen als Unter- bzw. Hinterfütterungen beispielsweise bei orthopädischen Schuhen bekannt (US-A-3 325 919). Diese kompakten Massen haben eine hohe Dichte von 1,1 bis 1,75, was sich bei verschiedenen Abformarbeiten sehr nachteilig auswirkt. Um das Verbleiben der schweren Masse an Ort und Stelle während des Aushärtens zu gewährleisten, müssen infolge der hohen Dichte entsprechende Hilfsvorrichtungen verwendet werden. Weiters bereitet es Schwierigkeiten, diese bekannten Massen, welche schlechte Fließeigenschaften aufweisen, mittels Druck beispielsweise durch einen dünnen Schlauch oder eine Kanüle in kurzer Zeit an eine bestimmte Stelle zu bringen.

Aus der DE-B-2 519 910 ist ferner ein Polster für Schuhe und orthopädische Gegenstände bekannt, dessen Füllung aus druckelastischen Mikrohohlkugeln gebildet ist, deren dünne Hülle aus Polyvinylidenchlorid besteht, wobei die Mikrohohlkugeln mittels eines vernetzten Polysulfides oder Polysioxans fest und elastisch miteinander verbunden sind. Die Polsterfüllung besteht also zum überwiegenden Teil aus lediglich an den Berührungspunkten miteinander verbundenen Mikrohohlkugeln, so daß diese Polsterfüllung eine schwammartige Struktur besitzt und eine geringe Dichte aufweist. Polster mit einer solchen Füllung passen sich langsam an eine bestimmte Form des abzustützenden Körpers an und es ist auch möglich, eine solche Anpassung beliebig oft an bestimmte Körperformen vorzunehmen, wobei diese Polster reversibel stauchbar sind und eine elastische Abstützung gewährleisten. Solche Polster sind also leicht und garantieren eine komfortable Abstützung, die Polsterfüllung ist jedoch als Masse, die zur Herstellung oder Unterfütterung von Zahnprothesen geeignet ist, nicht verwendbar, denn hier ist es erforderlich, daß die Masse sich rasch an die bestimmte Körperform anpaßt und die abgeformte Gestalt beibehält.

Die vorliegende Erfindung hat sich somit zur Aufgabe gestellt, eine solche Masse zur Herstellung oder Unterfütterung von Zahnprothesen zu schaffen, welche, vor allem auch unter Druck, hervorragende Fließeigenschaften aufweist, so daß sie leicht auch in kleine Kavitäten einzudringen vermag und den abzuformenden Teil genau nachformt, weiters eine gewisse Elastizität besitzt, so daß ein Abziehen der verfestigten Masse auch über vorspringende Teile möglich ist, jedoch die Form des abzuformenden Teiles genau beibehält. Die erfindungsgemäß verwendete Masse ist hierbei im wesentlichen dadurch gekennzeichnet, daß sich in ihr Mikrohohlkugeln in einer Menge zwischen 10 und 65 Vol.-%, vorzugsweise zwischen 20 und 50 Vol.-%, befinden.

Die erfindungsgemäß verwendete Masse besteht somit zum Großteil aus Silikon, dem jedoch die erwähnten Mikrohohlkugeln beigemengt sind, wodurch aus der niedrigviskosen Silikonmasse eine hochviskose, pastöse bzw. knetartige Masse mit geringer Dichte wird, so daß die Fließeigenschaften der erfindungsgemäßen Masse unter Druck wesentlich besser sind als bei den bisher verwendeten Massen. Dies wird wahrscheinlich dadurch bewirkt, daß die Mikrohohlkugeln eine außergewöhnlich glatte Oberfläche besitzen und dadurch die innere Reibung der Masse reduziert wird. Die erfindungsgemäß verwendete Masse verhält sich bei raschen Bewegungen somit mehr thixotrop als dilatant, so daß sie leicht auch in kleine Hohlräume einzudringen und daher feinste Strukturen abzuformen vermag. Diese Tatsache wird auch dadurch erklärt, daß beim Anpressen der Masse an abzuformende Körperteile die flüssigen Bestandteile der Masse an die Oberfläche gelangen, also diese Oberfläche durch niedrigviskose Silikonmasse angereichert wird. Es ist daher bei der erfindungsgemäßen Verwendung der Masse als Abdruckmasse für Zahnprothesen nicht mehr erforderlich, nach Anfertigung eines Vorabdruckes noch eine Feinabformung vorzunehmen. Die erfindungsgemäß verwendete Masse weist weiters die gewünschte Druck- und Zugelastizität auf, wobei sie nach ihrer Kondensations- oder Additionsvernetzung, ohne daß sie seitlich ausweichen kann, unter gleichen Bedingungen ihr Volumen unter Druck ähnlich verringern kann wie im flüssigen oder knetbaren Zustand und bei Druckentlastung sich spontan praktisch vollständig zurückstellt. Die Beimengung der Mikrohohlkugeln im erwähnten Ausmaß ermöglicht nämlich ein Komprimieren der erfindungsgemäßen Masse bis zu 50 % der volumsmäßig anteiligen Mikrohohlkugeln, ohne daß hiebei das in diesen Mikrohohlkugeln eingeschlossene Gas entweicht, so daß sich bei Druckentlastung auch bei der unvernetzten Masse das Volumen praktisch wieder zur Gänze auf das Ausgangsvolumen zurückstellt.

Ein weiterer Vorteil der erfindungsgemäß verwendeten Masse ist es, daß für eine bestimmte Aushärtezeit weniger Härter benötigt wird, da ja bei gleichem Volumen der Masse weniger Silikonanteil enthalten ist, und daß sich der Härter auch im knetbaren Zustand des Silikons leichter und schneller verteilen läßt, da, wie bereits erwähnt, durch die Mikrohohlkugeln die innere Reibung der Masse herabgesetzt wird. Zur Beimengung werden Mikrohohlkugeln üblichen Durchmessers zwischen 0,01 und 0,1 mm verwendet. Zweckmäßig ist es, wenn erfindungsgemäß die Mikrohohlkugeln verschiedenen Durchmesser aufweisen. Dadurch wird der Vorteil erzielt, daß unter Druck die größeren Mikrohohlkugeln von der Oberfläche zurückweichen und die kleineren Mikrohohlkugeln auch in enge und schmale Hohlräume einzudringen vermögen.

Die erfindungsgemäß verwendete Masse weist vorzugsweise eine Dichte zwischen 0,4 und 0,65 auf.

Bei der Herstellung der Masse kann nach einem bevorzugten erfindungsgemäßen Verfahren so vorgegangen werden, daß dem die Masse bildenden Silikon bzw. zumindest einem Teil dieses Silikons und/oder den Zuschlagstoffen bzw. zumindest einem Teil dieser Zuschlagstoffe ein Treibmittel enthaltende Kompaktteilchen aus Vinylidenchlorid-Copolymerisat beigemengt werden, wobei zumindest der Teil der Masse, dem die Kompaktteilchen beigemengt werden, unmittelbar vor oder nach der Beimengung auf eine Temperatur von über 80 °C, vorzugsweise zwischen 80° und 165 °C erhitzt wird, wobei sich die Mikrohohlkugeln in situ bilden. Die ein Treibmittel enthaltenden Kompaktteilchen aus Vinylidenchlorid-Copolymerisat sind hiebei unter der Bezeichnung EXPANCEL* im Handel erhältlich und können beispielsweise von der Fa. Kema-Nord in Sundvall/Schweden bezogen werden. Die daraus entstehenden Mikrohohlkugeln sind in der DE-OS-1 495 485 und in der Zeitschrift « Modern Plastics », August 1969, Seite 55 beschrieben. Dadurch, daß sich die Mikrohohlkugeln in der Masse in situ bilden, wird eine gleichmäßige Verteilung dieser Mikrohohlkugeln sichergestellt und es entstehen Mikrohohlkugeln verschiedenen Durchmessers, was den bereits erwähnten Vorteil mit sich bringt. Werden die Kompaktteilchen einem bereits über 80 °C erhitzten Teil der Masse hinzugefügt, so bilden sich die Mikrohohlkugeln besonders rasch. Auch dann, wenn die Masse erst nach dem Beimengen der Kompaktteilchen auf eine Temperatur über 80 °C erhitzt wird, ist es möglich, vor dem Beimengen eine Vorerwärmung des Teiles der Masse, dem die Kompaktteilchen beigemengt werden, durchzuführen, damit in der Folge die Erhitzung schneller durchgeführt werden kann.

Zweckmäßig ist es, zumindest den Teil der Masse, dem die Kompaktteilchen beigemengt werden, während der Erhitzung einem Druck auszusetzen. Wird in der Folge der Druck reduziert bzw. aufgehoben, so erfolgt eine Volumenzunahme der Masse und es wird deren Viskosität größer, wobei sich die Mikrohohlkugeln bilden und somit die Masse ihre geschlossenzellige Struktur erhält.

Die erfindungsgemäße Vorrichtung, mit welcher das Verfahren zur Herstellung der Masse kontinuierliche durchgeführt werden kann, weist einen Kanal mit vorzugsweise geringem Querschnitt, eine Fördereinrichtung zum Hindurchführen der mit den beigemengten Kompaktteilchen versehenen Masse durch den Kanal und eine Heizeinrichtung zum Erhitzen der im Kanal befindlichen Masse auf. Mit einer solchen Vorrichtung kann eine ununterbrochene Erzeugung der erfindungsgemäßen Masse durchgeführt werden, wobei es lediglich nötig ist, die mit den beigemengten Kompaktteilchen versehene Masse mittels der Fördereinrichtung durch den Kanal zu befördern und während der Verweildauer im Kanal auf die erforderliche Temperatur zu erhitzen, so daß beim Austreten aus dem Kanal die Mikrohohlkugeln bereits gebildet sind oder sich zumindest unmittelbar nach dem Austreten bilden.

Der Kanal kann erfindungsgemäß von einem Rohr gebildet sein, an dessen Austrittsende eine Düse vorgesehen ist. Durch diese Düse wird bewirkt, daß die Masse im Kanal unter Druck steht und nach dem Austreten aus der Düse die gewünschte, bereits erläuterte Volumenzunahme erfolgt, so daß die Masse von einer niedrigeren Viskosität zu einer höheren Viskosität gelangt.

Die Aufheizung der Masse im Kanal erfolgt zweckmäßig dadurch, daß die Heizeinrichtung mit der beispielsweise aus Metall bestehenden Kanalwandung in Verbindung steht. Diese Kanalwandung überträgt dann während des Transportes der Masse durch den Kanal ihre Wärme auf die Masse, so daß diese gleichmäßig erhitzt wird und spätestens beim Austreten aus dem Kanal die erforderliche Temperatur besitzt.

Die Fördereinrichtung kann beispielsweise von einer am Eintrittsende des Kanales angeordneten Förderschnecke gebildet sein. Bei einer Förderung der Masse durch den Kanal mittels einer solchen Förderschnecke werden unerwünschete Lufteinschlüsse weitgehend vermieden.

Die Erfindung wird im folgenden anhand eines Beispieles näher erläutert :

Eine fließfähige, anorganische Füllstoffe enthaltende Silikonmasse mit einer Viskosität von ca. 40 Pa · s (40 000 cP) und einer Dichte von 1,25 wird mit 6 Gew.-% ein Treibmittel enthaltender Kompaktteilchen aus Vinylindenchlorid-Copolymerisat, wie sie im Handel unter der Bezeichnung EXPANCEL erhältlich sind, und 9 Gew.-% reinem Paraffin vermischt und mittels einer Förderschnecke durch einen 75 cm langen Kanal transportiert, dessen Wandung durch eine Heizeinrichtung erhitzt wird. Am Austrittsende des Kanales ist eine Düse vorgesehen, über welche die Masse den Kanal mit einer Temperatur von ca. 110 °C verläßt. Nach dem Austreten der Masse aus der Düse expandiert die flüssige Masse und wird pastös bzw. knetbar. Die Masse weist dann eine Viskosität von mehr als 100 Pa · s (100 000 cP), gemessen im Brookfield-Viskosimeter (2V4, n = 6 U/min) und eine Dichte von ca. 0,55 auf. Die fertige Masse verhält sich ausgesprochen thixotrop und läßt sich unter Druck leicht verteilen. Zur Vernetzung im Mund ist, bezogen auf das Gesamtvolumen der Masse, weniger als die Hälfte der Härtermenge notwendig, welche bei einer bisher üblichen Masse notwendig wäre, welche keine Kompaktteilchen enthält.

Durch den Zusatz von Paraffin oder anderen ähnlichen inerten organischen Substanzen werden die Anpassungs- und Kneteigenschaften der geschlossenzelligen Masse verbessert.

Wird die erfindungsgemäß verwendete Masse zur Unterfütterung bzw. Auskleidung von Zahnprothesen, verwendet, so wird auf die zu unterfütternde bzw. auszukleidende Stelle der Prothese ein Silikonhaftvermittler aufgestrichen und darauf die erfindungsgemäße, noch nicht ausgehärtete Masse appliziert. Diese Masse wird dann an den betreffenden Kieferteil angedrückt und aushärten gelassen, so daß dadurch die Prothese genau an diesen Kieferteil angepaßt wird. Besonders vorteilhaft ist diese Vorgangsweise bei der Unterfütterung von Zahnprothesen, da hier durch Veränderung des Kiefers, Zahnfleischschwund und dgl. sehr häufig eine Anpassung einer bereits vorhandenen Zahnprothese vorgenommen werden muß.

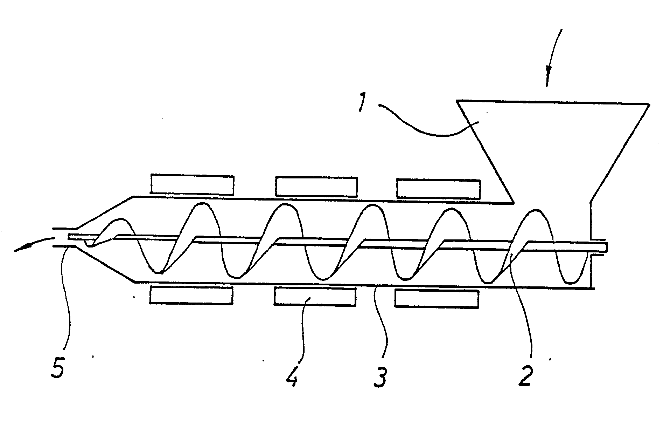

In der Zeichnung ist eine erfindungsgemäße Vorrichtung schematisch dargestellt.

Über einen Trichter 1 wird eine mit beigemengten, ein Treibmittel enthaltenden Kompaktteilchen aus Vinylidenchlorid-Copolymerisat sowie gegebenenfalls mit Zuschlagstoffen versehene Masse aus Silikon einer Förderschnecke 2 zugeführt, die diese Masse durch einen von einem metallischen Rohr 3 begrenzten Kanal hindurchfördert. An der Aussenseite des Rohres 3 sind Heizkörper 4 angeordnet, durch welche die in dem durch das Rohr 3 begrenzten Kanal befindliche Masse auf eine Temperatur über 80 °C erwärmt wird. Dadurch bilden sich aus den Kompaktteilchen in der Masse Mikrohohlkugeln, deren dünne Hülle aus Vinylidenchlorid-Copolymerisat besteht und deren Inneres ein Gas enthält.

An dem den Trichter 1 gegenüberliegenden Austrittsende des Rohres 3 ist eine Düse 5 vorgesehen, über welche die Masse austritt. Durch diese Düse 5 wird das Austreten der Masse aus dem Rohr verzögert, so daß diese Masse in dem vom Rohr 3 begrenzten Kanal unter Druck steht und nach dem Austreten eine Volumszunahme der Masse erfolgt, wodurch deren Viskosität vergrößert wird.

**Patentansprüche**

1. Verwendung von Massen bestehend aus einem Silikon, das zunächst flüssig oder pastös bzw. knetbar sich durch Zusetzen eines Härters nach einer bestimmten Zeit kautschukartig verfestigt, und aus dem Silikon beigemengten druckelastischen Mikrohohlkugeln, deren dünne aus Vinylidenchlorid-Copolymerisat besteht und deren Inneres ein Gas enthält, zur Herstellung oder Unterfütterung von Zahnprothesen, dadurch gekennzeichnet, daß sich in der Masse Mikrohohlkugeln in einer Menge zwischen 10 und 65 Vol.-%, vorzugsweise zwischen 20 und 50 Vol.-% befinden.

2. Verwendung von Massen nach Anspruch 1, dadurch gekennzeichnet, daß die Mikrohohlkugeln einen verschieden großen Durchmesser aufweisen.

3. Verwendung von Massen nach Anspruch 1, gekennzeichnet durch eine Dichte zwischen 0,4 und 0,65.

4. Verfahren zur Herstellung einer Masse nach einem der Ansprüche 1 bis 3, dadurch gekennzeichnet, daß dem die Masse bildenden Silikon bzw. zumindest einem Teil dieses Silikons und/ oder den Zuschlagstoffen bzw. zumindest einem Teil dieser Zuschlagstoffe ein Treibmittel enthaltende Kompaktteilchen aus Vinylidenchlorid-Copolymerisat beigemengt werden, wobei zumindest der Teil der Masse, dem die Kompaktteilchen beigemengt werden, unmittelbar vor oder nach der Beimengung auf eine Temperatur über 80 °C, vorzugsweise zwischen 80° und 165 °C, erhitzt wird, wobei sich die Mikrohohlkugeln in situ bilden.

5. Verfahren nach Anspruch 4, dadurch gekennzeichnet, daß zumindest der Teil der Masse, dem die Kompaktteilchen beigemengt werden, während der Erhitzung einem Druck ausgesetzt wird.

6. Vorrichtung zur Durchführung des Verfahrens nach Anspruch 4 oder 5, gekennzeichnet durch einen Kanal mit vorzugsweise geringem Querschnitt, eine Fördereinrichtung (2) zum Hindurchführen der mit den beigemengten Kompaktteilchen versehenen Masse durch den Kanal, und mit einer Heizeinrichtung (4) zum Erhitzen der im Kanal befindlichen Masse.

7. Vorrichtung nach Anspruch 6, dadurch gekennzeichnet, daß der Kanal von einem Rohr (3) gebildet ist, an dessen Austrittsende eine Düse (5) vorgesehen ist.

8. Vorrichtung nach Anspruch 6 oder 7, dadurch gekennzeichnet, daß die Heizeinrichtung (4) mit der beispielsweise aus Metall bestehenden Kanalwandung zwecks Aufheizung derselben in Verbindung steht.

9. Vorrichtung nach Anspruch 6, 7 oder 8, dadurch gekennzeichnet, daß die Fördereinrichtung (2) von einer am Eintrittsende des Kanales angeordneten Förderschnecke gebildet ist.

## Claims

1. Use of masses consisting of a silicone, which is first liquid or pasty or, respectively, kneadable and which solidifies in a certain time interval in a rubber-like manner by adding a hardener, and of pressure-elastic hollow microspheres added to the silicone and having their thin shell consisting of a vinylidene chloride copolymer and containing within their interior a gas, for the production of or for lining teeth prostheses, characterized in that there are present within the mass hollow microspheres in an amount between 10 and 65 percent by volume, preferably between 20 and 50 percent by volume.

2. Use of masses as claimed in claim 1, characterized in that the hollow microspheres have a diameter of different magnitude.

3. Use of masses as claimed in claim 1, characterized by a density between 0.4 and 0.65.

4. Process for producing a mass according to any of the claims 1 to 3, characterized in that compact particles consisting of a vinylidene chloride copolymer and containing an inflating agent are added to the silicone forming the mass or, respectively, to at least part of this silicone and/or to the additives or, respectively, to at least part of these additives, noting that at least part of the mass, to which are added the compact particles, is heated immediately prior to said addition or after said addition to a temperature above 80 °C, preferably between 80 and 165 °C, whereby the hollow microspheres are formed in situ.

5. Process as claimed in claim 4, characterized in that at least that part of the mass, to which are added the compact particles, is subjected to pressure during heating.

6. Apparatus for performing the process according to claim 4 or 5, characterized by a channel of preferably small cross section, and conveyor means (2) for passing through the channel the mass provided with the added compact particles and comprising a heating means (4) for heating the mass being present within the channel.

7. Apparatus as claimed in claim 6, characterized in that the channel is formed of a tube (3) having provided a nozzle (5) at its exit end. .

8. Apparatus as claimed in claim 6 or 7, characterized in that the heating means (4) is in contact with the channel wall, for example consisting of metal, for the purpose of heating said wall.

9. Apparatus as claimed in claim 6, 7 or 8, characterized in that the conveyor means (2) is formed of a conveyor screw arranged at the entry end of the channel.

## Revendications

1. Utilisation de masses constituées d'un silicone qui, tout d'abord liquide ou pateux, ou bien modelable, se solidifie au bout d'un temps déterminé à la façon d'un caoutchouc par addition d'un durcisseur, et de microbilles creuses élastiques à la compression, mélangées au silicone et dont l'enveloppe mince est constituée d'un copolymérisat de chlorure de vinylidène et dont l'intérieur contient un gaz, pour la fabrication ou le revêtement de prothèses dentaires, caractérisé en ce que, dans la masse, des micro-billes creuses sont présentes en une quantité entre 10 et 65 Vol.-%, et de préférence entre 20 et 50 Vol.-%.

2. Utilisation de masses selon la revendication 1, caractérisée en ce que les micro-billes creuses présentent des diamètres de taille différente.

3. Utilisation de masses selon la revendication 1, caractérisée par une densité entre 0,4 et 0,65.

4. Procédé de séparation d'une masse selon l'une des revendications 1 à 3, caractérisé en ce que l'on mélange au silicone formant la masse ou au moins une partie de ce silicone et/ou aux matières d'adjonction, ou au moins une partie de ces matières d'adjonction, des particules compactes en copolymérisat de chlorure de vinylidène contenant un agent pousseur, la partie, au moins de la masse, à laquelle sont mélangées les particules compactes, étant, immédiatement avant ou après le mélange, chauffée à une température de 80° ou plus, de préférence entre 80° et 165 °C, les micro-billes creuses se formant in situ.

5. Procédé selon la revendication 4, caractérisé en ce que au moins la partie de la masse à laquelle les particules compactes sont mélangées, est soumise à une pression pendant le chauffage.

6. Dispositif pour la mise en œuvre du procédé selon la revendication 4 ou 5, caractérisé par un canal avec une section transversale de préférence faible, un dispositif de transport (2) pour conduire la masse pourvue des particules mélangées, à travers le canal, et un dispositif de chauffage (4) pour chauffer la masse qui se trouve dans le canal.

7. Dispositif selon la revendication 6, caractérisé en ce que le canal est formé d'un tube (3) à l'extrémité de sortie duquel est prévu un ajustage (5).

8. Dispositif selon la revendication 6 ou 7, caractérisé en ce que le dispositif de chauffage (4) est en liaison, pour son chauffage, avec la paroi de canal constituée par exemple en métal.

9. Dispositif selon la revendication 6, 7 ou 8, caractérisé en ce que le dispositif de transport (2) est formé d'une vis transporteuse disposée à l'extrémité d'entrée du canal.